# EUROPEAN PATENT SPECIFICATION

(11) **EP 3 447 145 B1**
(45) Date of publication and mention of the grant of the patent: **11.05.2022**
(21) Application number: 16909598.1
(22) Date of filing: 15.11.2016
(51) Int. Cl.: C12Q 1/68, C12Q 1/6848

(54) **TARGET NUCLEIC ACID SEQUENCE DETECTION METHOD USING MULTIPLE AMPLIFICATION NESTED SIGNAL AMPLIFICATION**
VERFAHREN FÜR DEN NACHWEIS VON ZIELNUKLEINSÄURESEQUENZEN ANHAND VON VERSCHACHTELTER SIGNALVERSTÄRKUNG MIT MEHRFACHVERSTÄRKUNG
MÉTHODE DE DÉTECTION DE SÉQUENCE D'ACIDE NUCLÉIQUE CIBLE UTILISANT UNE AMPLIFICATION NICHÉE DE SIGNAL DE TYPE AMPLIFICATION MULTIPLE

(30) Priority: 20.07.2016 KR 20160091960
(43) Date of publication of application: 27.02.2019
(73) Proprietor: Diogene Co., Ltd, Seongnam-si, Gyeonggi-do 13486 (KR)
(72) Inventor: LIM, Seong Sik, Namyangju-si Gyeonggi-do 12185 (KR); KIM, Yeon Soo, Namyangju-si Gyeonggi-do 12185 (KR)
(74) Representative: Isarpatent
(86) International application number: PCT/KR2016/013136
(87) International publication number: WO 2018/016683

(56) References cited:
- WO-A1-2006/095981
- WO-A1-2007/025340
- WO-A1-2011/078439
- JP-B2- 4 517 061
- KR-A- 20080 052 626
- KR-A- 20150 056 151
- KR-A- 20150 109 478
- KR-A- 20160 052 400
- STANLEY et al.: "Multiplexed Tandem PCR: Gene Profiling from Small Amounts of RNA Using SYBR Green Detection", Nucleic Acids Research, vol. 33, no. 20, 2005, page e180, XP003009144,

<110> DIOGENE INC.
<120> Method for detection of target nucleic acid sequence by multiple amplification nested signal amplification
<130> P16-1525
<160> 3
<170> KoPatentIn 3.0
<210> 1
   <211> 210
   <212> DNA
   <213> Artificial Sequence
<220>
   <223> genomic DNA of zika virus
<400> 1
   gggaagttca acgagccaaa aagtcatata tctggtcatg atactgctga ttgccccggc 60
   atacagcatc aggtgcatag gagtcagcaa tagggacttt gtggaaggta tgtcaggtgg 120
   gacttgggtt gatgttgtct tggaacatgg aggttgtgtt accgtaatgg cacaggacaa 180
   accgactgtc gacatagagc tggttacaac 210
<210> 2
   <211> 29
   <212> DNA
   <213> Artificial Sequence
<220>
   <223> Synthetic Sequence
<220>
   <221> misc_feature
   <222> (6)..(8)
   <223> n is deoxyinosine
<400> 2
   gcaatnnngg tcatgatact gctgattgc 29
<210> 3
   <211> 26
   <212> DNA
   <213> Artificial Sequence
<220>
   <223> Synthetic Sequence
<220>
   <221> misc_feature
   <222> (6)..(8)
   <223> n is deoxyinosine
<400> 3
   gcaatnnncc acaaagtccc tattgc 26

## Description

### Technical Field

The present invention relates to a method of detecting a target nucleic acid sequence using multiple amplification nested signal amplification (MANSA), and more particularly to a method of amplifying and detecting a target nucleic acid sequence through a primary amplification reaction and a secondary signal amplification reaction of the target nucleic acid sequence using a dumbbell-structure oligonucleotide.

### Background Art

Currently, the most commonly used method for obtaining a gene sample is a polymerase chain reaction method using a DNA polymerase. Oligonucleotides used for the polymerase chain reaction are designed to bind to the opposite strands of template DNA. This method has an advantage in that only the desired region of the target gene may be amplified accurately by arbitrarily adjusting the length and base sequence of the oligonucleotide capable of binding to the target DNA, but is disadvantageous because only one target gene may be amplified through a single reaction, and thus when the number of target genes to be amplified is large, the same procedures must be repeated.

In order to solve these drawbacks, methods for performing polymerase chain reaction (PCR) by mixing two or more target genes and primers corresponding to respective target genes are being developed, and also, many methods have been developed to improve the annealing specificity of primers. Examples thereof include touch-down PCR (Don et al., 1991), hot-start PCR (D'Aquila et al., 1991), nested PCR (Mullis and Faloona, 1987) and booster PCR (Ruano et al., 1989). Another approach is to improve the specificity of PCR using a variety of enhancer compounds. Here, the enhancer compounds include chemicals that increase the effective binding reaction temperature, DNA binding proteins, commercially available reactants, and the like. However, successful results cannot be obtained in all PCR methods, and testing these additives under various binding temperature conditions requires much time and effort. The approaches described above contribute somewhat to improving the primer-annealing specificity, but are not a fundamental solution to problems resulting from the primers used for PCR amplification, such as nonspecific products and high background. Moreover, the number of genes that may be successfully amplified at one time is only three or four, and drawbacks, such as competition or interference effects among the genes, and amplification of nonspecific products, have not been overcome, and thus the above methods are not yet used in practice.

In order to perform multiplex PCR and allelespecific PCR, the conditions of a target gene of interest must be optimized. To this end, lots of time, effort, and sample consumption are required, and the conditions thus optimized do not apply to other genes. With the goal of overcoming these problems, methods such as linker PCR or ligation-mediated PCR (Journal of Clinical Microbiology, 43(11):5622-5627, 2005) have been developed. However, in the linker PCR, cross-contamination may occur due to the experimental procedure of transferring some of the reaction product in the first tube to the second tube, and in ligation-mediated PCR, it is difficult for researchers to perform complex experimental methods using various kinds of enzymes, and such experimental methods are partially employed.

Accordingly, there has been demand for development of a technique capable of realizing gene amplification in an inexpensive and simple manner. The technique developed to meet this demand is to simply manipulate the primer so that amplification does not occur until the PCR reaction temperature is reached, which is exemplified by the invention disclosed in Korean Patent No. 649165. In this technique, a regulator site is additionally inserted into the initial primer. The regulator site is a polydeoxyinosine linker, and inosine constituting the regulator site is a universal base having low Tm compared to general nucleotides G, A, T and C, which typically constitute a primer, and the polydeoxyinosine linker forms a bubble-like structure at a specific temperature to thus block the nonspecific binding of the primer to the target, thereby suppressing the nonspecific amplification in PCR. This technique is somewhat inexpensive compared to the aforementioned conventional techniques, but is problematic in that the temperature of the binding step in the first cycle (the temperature suitable for the PCR) and the temperature of the binding step in the second cycle during the PCR have to be set differently from each other. This is because the sequences additionally introduced into the primers may participate in priming from the second PCR cycle. The application of different temperatures is not strictly required, but may be necessary for efficient PCR. Furthermore, a limitation is imposed on the above technique in which a pre-selected arbitrary nucleotide sequence at the 5'-end portion should be added and should not be complementary to any position of the target, which results in additional inconvenience. Furthermore, it is impossible to be sure of the success of application of the technique when all the gene sequences of the target are unknown. Hence, there is a need to develop a new method which is cheaper and easier to realize than the conventional technique.

Real-time PCR is widely used because amplification products are measured in real time and because more accurate quantitative analysis is possible. The conventional patent documents regarding real-time PCR include U.S. Patent Nos. 5,210,015, 5,538,848 and 6,326,145. The conventional real-time PCR methods are advantageous because of a homogeneous assay in which amplification and detection are performed simultaneously, but the number of target nucleic acid sequences that may be detected simultaneously is limited due to limitations of the kinds of fluorogenic reporter molecules. Since the existing thermocycler for real-time detection of the target nucleic acid sequence enables simultaneous detection up to 5-plex capacity, the number of target nucleic acid sequences that may be detected simultaneously is limited, and a long time and an additional expensive real-time monitoring device are required in order to analyze large volumes of samples.

The TaqMan probe method (U.S. Patent No. 5,210,015) and the self-quenching fluorescence probe method (U.S. Patent No. 5,723,591), which are representative real-time PCR methods, are problematic in false-positive results occur due to nonspecific binding of dual-labeled probes, making it difficult to actually realize the 5-plex detection. To this end, skilled technology and know-how are essential. Since the conventional real-time PCR method requires simultaneous amplification and detection, high throughput of a real-time PCR device is limited. WO-2011/078439 describes a method for detecting a target nucleic acid.

WO-2006/095981 describes a dual specificity oligo nucleotide of a general formula 5'-Xp-Yq-Zr-3'.

WO-2007/025340 describes a multiplex nested amplification reaction for detecting a TNA.

### Disclosure

### Technical Problem

Accordingly, the present disclosure provides a method of amplifying and detecting a target nucleic acid sequence, which enables hot-start PCR by inhibiting unintended PCR amplification at room temperature.

Another objective of the present disclosure is to provide a method of amplifying and detecting a target nucleic acid sequence, which is capable of suppressing nonspecific amplification in PCR as a result of predominating amplification from a PCR product compared to amplification from an original template upon PCR amplification.

Still another objective of the present disclosure is to provide a method of detecting multiple target nucleic acid sequences, which is capable of simultaneously detecting multiple target nucleic acid sequences using a multiplexing process and also of detecting multiple target nucleic acid sequences at improved sensitivity using a probe and a sample in very small amounts compared to conventional real-time PCR methods.

### Technical Solution

In order to accomplish the above objectives, the present invention provides a method of detecting a target nucleic acid sequence using a dumbbell-structure oligonucleotide according to claim 1, comprising the following steps of:

(a) amplifying a target nucleic acid sequence through a primary amplification reaction of a polymerase chain reaction in which a primer pair having a dumbbell-structure oligonucleotide represented by General Formula below is used, annealing is carried out in a temperature range of 50 °C to 65 °C, and a template-dependent extension reaction process, including hybridization, extension, and denaturation using the dumbbell-structure oligonucleotide, is repeated:
General Formula: 5'-A-B-C-3'
wherein A is a 5'-low Tm specificity portion having a nucleotide sequence that is complementary to one position of the target nucleic acid sequence to be hybridized, and includes a nucleotide sequence complementary to one position of C, B is a separation portion including at least three universal bases, C is a 3'-high Tm specificity portion having a nucleotide sequence that is complementary to one position of the target nucleic acid sequence to be hybridized, and includes a nucleotide sequence complementary to one position of A, Tm of A is 10 °C to 30 °C and is lower than Tm of C that is 50 °C to 65 °C, Tm of the separation portion is 3 °C to 10 °C that is the the lowest Tm among three portions, the separation portion forms a non-base pair structure under the condition that A and C are hybridized to the target nucleic acid sequence, the 5'-low Tm specificity portion is shorter than the 3'-high Tm specificity portion, the 5'-low Tm specificity portion has a length of 3 to 5 nucleotides, the separation portion has a length of 3 to 5 nucleotides, the 3'-high Tm specificity portion has a length of 18 to 30 nucleotides, and the 3'-high Tm specificity portion has a nucleotide sequence fully complementary to the target nucleic acid sequence to be hybridized, hybridization being carried out under the condition that hybridization by C alone does not occur; and
(b) amplifying the primary amplification product through a secondary amplification reaction using a primer pair including an oligonucleotide with the same dumbbell-structure as used in step (a) and a fluorogenic reporter including SYBR Green, SYTO 9, EvaGreen, or LCGreen and detecting a signal from the fluorogenic reporter, in which detection of the signal indicates the presence of the target nucleic acid sequence by analysis of a melting curve generated with a resolution ranging from 0.05 °C to 0.02 °C.

### Advantageous Effects

The method of the present invention can inhibit unintended PCR amplification at room temperature to thus enable hot-start PCR, and furthermore, can suppress nonspecific amplification in PCR as a result of predominating amplification from a PCR product compared to amplification from an original template upon PCR amplification.

Also, the method of the present invention uses a dumbbell-structure oligonucleotide as a primer, whereby many different genes can be simultaneously amplified in a rapid and accurate manner through a single PCR.

Moreover, according to the present invention, multiple target nucleic acids can be simultaneously detected through a multiplexing process. Here, multiple target nucleic acid sequences can be classified and distinguished depending on the purpose of analysis, and multiple target nucleic acid sequences can be detected at improved sensitivity using a probe and a sample in very small amounts compared to conventional real-time PCR methods.

Furthermore, in the method of the present invention, the presence or absence of mutation of various cancer genes or inhibitory genes depending on multiple amplification nested signal amplification and a difference in a melting temperature of a melting curve, without conventional additional restriction enzyme treatment or base sequence analysis, can be accurately detected at the same time without error.

The conventional real-time PCR methods require a complex modified primer structure such as a labeled probe or a hairpin structure, which makes it difficult to design, synthesize, and select the sequence of the probe and primer. However, the primer of the present invention enables real-time PCR to be carried out in a simple and easy manner because it is used for target amplification and signal amplification, without additional labeled probes or complex modified primer structures.

The conventional real-time PCR methods are not suitable for a multiplex assay because they make it difficult to design and optimize primers or probes. In contrast, the present invention can accurately detect the target nucleic acid sequence depending on the target amplification nested signal amplification and the difference in melting temperature of the melting curve upon real-time PCR, without additional probes or complex modified primer structures.

Thus, the method of the present invention is regarded as time- and cost-effective in the development of real-time PCR assays.

### Description of Drawings

FIG. 1 schematically shows a process of amplifying and detecting five target nucleic acid sequences using multiple amplification nested signal amplification according to the present invention.

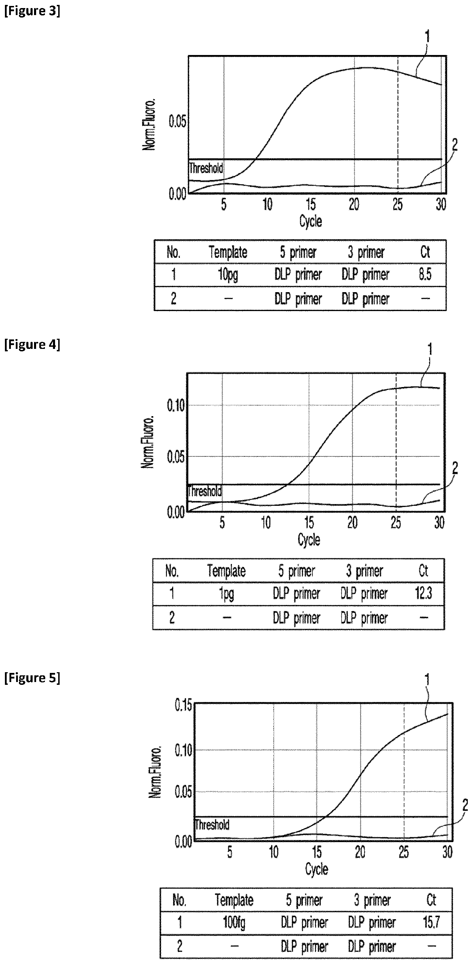

FIGS. 2 to 7 show the results of amplification (amplification curves) of individual target nucleic acid sequences at various concentrations using the process of the present invention;
FIG. 8 shows the results of amplification (amplification curves) using the process of the present invention after mixing target nucleic acid sequences at various concentrations;
FIG. 9 shows the results of electrophoretic analysis after amplification of target nucleic acid sequences at various concentrations;
FIG. 10 shows the results of melting curve analysis after amplifying a mixture of target nucleic acid sequences at various concentrations using the process of the present invention; and

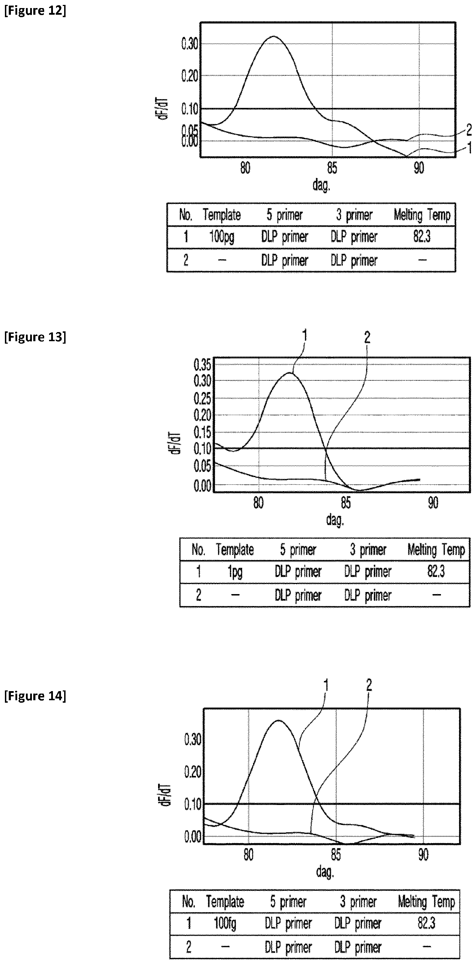

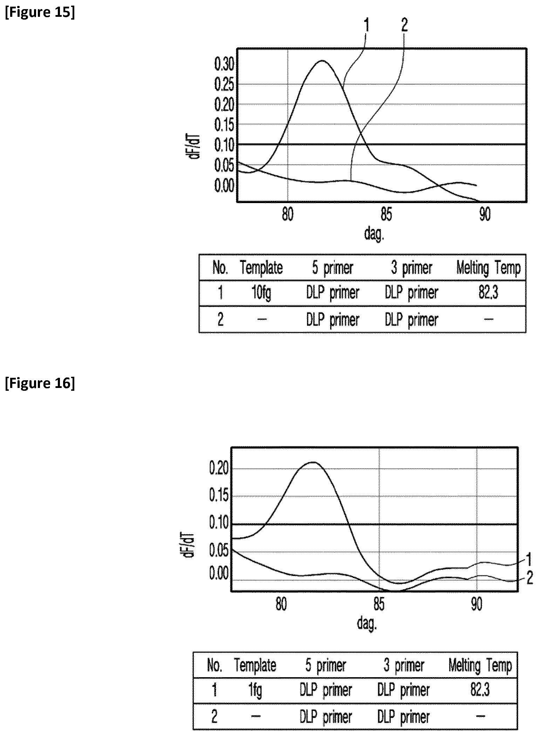

FIGS. 11 to 16 shows the results of melting curve analysis after amplifying each of target nucleic acid sequences at various concentrations using the process of the present invention.

### Best Mode

The present invention as defined by the appended claims pertains to a method of detecting a target nucleic acid sequence, which enables hot-start PCR by inhibiting unintended PCR amplification at room temperature and also which may suppress nonspecific amplification in PCR as a result of predominating amplification from a PCR product compared to amplification from an original template upon PCR amplification.

The present inventors have thoroughly studied methods capable of amplifying multiple genes through a single PCR and thus have ascertained that, upon primer formation for a gene base sequence to be amplified, when using a primer composed of a dumbbell-structure oligonucleotide configured such that a 5'-end is added with any base sequence for complementary binding to a 3'-end of 3-5 bp and a universal base pair of 3-5 bp for connecting these two portions, many different genes may be simultaneously amplified in a rapid and accurate manner through a single PCR. The present inventors have developed a technique in which multiplex target amplification is performed using the dumbbell-structure oligonucleotide to obtain an amplification product and then a target nucleic acid sequence is detected through the nested signal amplification.

Accordingly, the present invention provides a method of detecting a target nucleic acid sequence from a DNA or nucleic acid mixture using multiple amplification nested signal amplification in a liquid phase, which will be made clearer by the examples and claims, which are to be described later.

An aspect of the present invention pertains to a method of detecting a target nucleic acid sequence using a dumbbell-structure oligonucleotide according to claim 1, comprising the following steps of:

(a) amplifying a target nucleic acid sequence through a primary amplification reaction of a polymerase chain reaction in which a primer pair having a dumbbell-structure oligonucleotide represented by General Formula below is used, annealing is carried out in a temperature range of 50 °C to 65 °C, and a template-dependent extension reaction process, including hybridization, extension, and denaturation using the dumbbell-structure oligonucleotide, is repeated:
General Formula: 5'-A-B-C-3'
wherein A is a 5'-low Tm specificity portion having a nucleotide sequence that is complementary to one position of the target nucleic acid sequence to be hybridized, and includes a nucleotide sequence complementary to one position of C, B is a separation portion including at least three universal bases, C is a 3'-high Tm specificity portion having a nucleotide sequence that is complementary to one position of the target nucleic acid sequence to be hybridized, and includes a nucleotide sequence complementary to one position of A, Tm of A is 10 °C to 30 °C and is lower than Tm of C that is 50 °C to 65 °C, Tm of the separation portion is 3 °C to 10 °C that is the the lowest Tm among three portions, the separation portion forms a non-base pair structure under the condition that A and C are hybridized to the target nucleic acid sequence, the 5'-low Tm specificity portion is shorter than the 3'-high Tm specificity portion, the 5'-low Tm specificity portion has a length of 3 to 5 nucleotides, the separation portion has a length of 3 to 5 nucleotides, the 3'-high Tm specificity portion has a length of 18 to 30 nucleotides, and the 3'-high Tm specificity portion has a nucleotide sequence fully complementary to the target nucleic acid sequence to be hybridized, hybridization being carried out under the condition that hybridization by C alone does not occur; and
(b) amplifying the primary amplification product through a secondary amplification reaction using a primer pair including an oligonucleotide with the same dumbbell-structure as used in step (a) and a fluorogenic reporter including SYBR Green, SYTO 9, EvaGreen, or LCGreen and detecting a signal from the fluorogenic reporter, detection of the signal indicating the presence of the target nucleic acid sequence by analysis of a melting curve generated with a resolution ranging from 0.05 °C to 0.02 °C.

The method of the present invention includes (a) primary amplification reaction of a target nucleic acid sequence in a biosample; and (b) secondary amplification reaction (signal amplification) of the primary amplification product and signal detection.

According to the present invention, only the target nucleic acid sequence is amplified through the primary amplification reaction, and then the resulting amplification product is used as a template to amplify the signal. Compared to the conventional techniques, the present invention is characterized in that signal amplification and real-time monitoring by exonuclease activity are possible through primary amplification of only the target nucleic acid sequence and then secondary amplification of the primary amplification product (signal amplification is simultaneous therewith), which is referred to as "multiple amplification nested signal amplification" in the present invention, and abbreviated as "MANSA".

Multiplex amplification of the target nucleic acid may be performed in accordance with various primermediated nucleic acid amplification methods known in the art. Preferably, amplification of the target nucleic acid is carried out by PCR, and the PCR method is disclosed in U.S. Patent Nos. 4,683,195, 4,683,202 and 4,800,159. The target nucleic acid sequence that is subjected to multiplex amplification is DNA or RNA. The nucleic acid molecule may be double-stranded or single-stranded, and is preferably double-stranded. When the nucleic acid serving as the starting material is double-stranded, it is preferable to divide the two strands into a single strand or into a partial single strand. Methods of separating the strands include, but are not limited to, treatment with heat, alkali, formamide, urea and glyoxal, enzymatic methods (e.g., helicase) and binding protein treatment. For example, strand separation may be achieved through heat treatment at 80 to 105°C. When mRNA is used as a starting material, a reverse transcription step is required before amplification, and in the reverse transcription step, an oligonucleotide dT primer hybridizable to poly A tail of mRNA, a random hexamer, or an oligonucleotide complementary to a target nucleic acid sequence may be used. The oligonucleotide dT primer consists of dTMPs, and one or more of the dTMPs may be replaced with other dNMPs, so long as the dT primer may act as a primer. The reverse transcription step may be carried out using a reverse transcriptase having RNase H activity. When the enzyme having RNase H activity is used, careful selection of the reaction conditions may avoid individual RNase H cleavage processes.

As used herein, the term "oligonucleotide" refers to a linear oligomer of natural or modified monomers or linkages, includes deoxyribonucleotides and ribonucleotides, may be specifically hybridized to the target nucleotide sequence, and may naturally occur or may be artificially synthesized. The oligonucleotide is preferably single-stranded in order to realize maximum hybridization efficiency. Preferably, the oligonucleotide is oligodeoxyribonucleotide. The oligonucleotide of the present invention may include naturally occurring dNMPs (i.e. dAMP, dGMP, dCMP and dTMP), nucleotide analogs or derivatives. Also, the oligonucleotide may include ribonucleotide. For example, the oligonucleotide of the present invention may include backbone-modified nucleotides, for example, peptide nucleic acid (PNA) (M. Egholm et al., Nature, 365:566-568(1993)), phosphorothioate DNA, phosphorodithioate DNA, phosphoramidate DNA, amide-linked DNA, MMI-linked DNA, 2'-O-methyl RNA, alpha-DNA and methylphosphonate DNA, sugar-modified nucleotides, for example, 2'-O-methyl RNA, 2'-fluoro RNA, 2'-amino DNA, 2'-O-alkyl DNA, 2'-O-allyl DNA, 2'-O-alkynyl DNA, hexose DNA, pyranosyl RNA and anhydrohexitol DNA, and base-modified nucleotides, for example, C-5 substituted pyrimidine (where the substituent includes fluoro-, bromo-, chloro-, iodo-, methyl-, ethyl-, vinyl-, formyl-, ethynyl-, propynyl-, alkynyl-, thiazolyl-, imidazolyl-, and pyridyl-), C-7 substituted 7-deazapurine (where the substituent includes fluoro-, bromo-, chloro-, iodo-, methyl-, ethyl-, vinyl-, formyl-, alkynyl-, alkenyl-, thiazolyl-, imidazolyl-, and pyridyl-), inosine and diaminopurine.

As used herein, the term "primer" refers to an oligonucleotide, and may serve as an initiation point for synthesis under conditions of inducing the synthesis of a primer extension product complementary to the nucleic acid chain (template), i.e. under conditions of the presence of a nucleotide polymerization agent such as a DNA polymerase, and a suitable temperature and pH. In order to realize maximum amplification efficiency, the primer is preferably single-stranded. Preferably, the primer is deoxyribonucleotide. The primer of the present invention may include naturally occurring dNMPs (i.e. dAMP, dGMP, dCMP and dTMP), modified nucleotides or non-naturally occurring nucleotides. Also, the primer may include a ribonucleotide.

The primer should be long enough to be able to prime the synthesis of the extension product in the presence of the polymerization agent. The appropriate length of the primer is determined by a number of factors, such as temperature, application, and source of the primer.

As used herein, the term "annealing" or "priming" refers to apposition of oligodeoxynucleotide or nucleic acid to the template nucleic acid, and the apposition causes the polymerase to polymerize the nucleotide to form a nucleic acid molecule complementary to the template nucleic acid or to a part thereof. As used herein, the term "hybridization" refers to formation of a double-stranded nucleic acid from a complementary single-stranded nucleic acid. The terms "annealing" and "hybridization" are not different, and are used interchangeably herein.

As used herein, the term "probe" refers to a single-stranded nucleic acid molecule that contains a substantial complementary portion of the target nucleotide sequence.

Used in the present invention, the dumbbell-structure oligonucleotide is hybridized (annealed) to the target nucleic acid sequence to thus amplify the target nucleic acid sequence, and has the structure represented by the following General Formula, as also defined in claim 1.

General Formula: 5'-A-B-C-3'

Here, A is a 5'-low Tm specificity portion having a nucleotide sequence that is substantially complementary to one position of the target nucleic acid sequence to be hybridized, and includes a nucleotide sequence substantially complementary to one position of C, B is a separation portion including at least three universal bases, C is a 3'high Tm specificity portion having a nucleotide sequence that is substantially complementary to one position of the target nucleic acid sequence to be hybridized, and includes a nucleotide sequence substantially complementary to one position of A, Tm of A is lower than Tm of C, the separation portion has the lowest Tm among three portions, and the separation portion forms a non-base pair structure under the condition that A and C are hybridized to the target nucleic acid sequence, the hybridization being carried out under the condition that hybridization by C alone does not occur.

Used in the present invention, the dumbbell-structure oligonucleotide may exhibit significantly improved hybridization specificity because hybridization is determined in a type-specific manner by the 5'-low Tm specificity portion and the 3'-high Tm specificity portion separated by the separation portion.

More particularly, when the dumbbell-structure oligonucleotide is annealed to the target nucleic acid sequence, the specificity is not determined by the total length of the primer, as in conventional primers, but is doubly determined by the 5'-low Tm specificity portion and the 3'-high Tm specificity portion, which are separated from each other. Thus, when the dumbbell-structure oligonucleotide is annealed to the target nucleic acid sequence, it is annealed in a different manner compared to conventional primers, resulting in a significant improvement in annealing specificity.

In the dumbbell-structure oligonucleotide used in the present invention, the universal base located in the separation portion is selected from the group consisting of deoxyinosine, inosine, 7-diaza-2'-deoxyinosine, 2-aza-2'-deoxyinosine, 2'-OMe inosine, 2'-F inosine and combinations of the above bases, and is preferably deoxyinosine, inosine, or 5-nitroindole, and is most preferably deoxyinosine. According to a preferred embodiment of the present invention, the separation portion includes contiguous universal bases.

In the dumbbell-structure oligonucleotide used in the present invention, the length of the 5'-low Tm specificity portion is shorter than the length of the 3'-high Tm specificity portion. The 5'-low Tm specificity portion has a length of, 3 to 15 nucleotides which is described and not claimed *per se,* or 3 to 5 nucleotides which is according to the present invention. The 3'-high Tm specificity portion has a length of 3 to 30 nucleotides, 5 to 19 nucleotides, or 6 to 18 nucleotides which is described and not claimed *per se,* or 18 to 30 nucleotides which is according to the present invention. The separation portion has a length of 3 to 5 nucleotides. According to the present invention, the 5'-low Tm specificity portion has a length of 3 to 5 nucleotides, the separation portion has a length of 3 to 5 nucleotides, and the 3'-high Tm specificity portion has a length of 18 to 30 nucleotides.

According to the present invention, the 5'-low Tm specificity portion has Tm of 10 to 30°C. Also, the 3'-high Tm specificity portion has Tm of 50 to 65°C. The separation portion preferably has Tm of 3 to 10°C.

According to the present invention, the 3'-high Tm specificity portion has a nucleotide sequence fully complementary to the target nucleic acid sequence to be hybridized.

According to the present invention, the annealing of the dumbbell-structure oligonucleotide to the target nucleic acid sequence is performed in the temperature range of 50°C to 65°C.

According to the present inventon, primary amplification of the target nucleic acid sequence is implemented by repeating a template-dependent extension reaction process, including the hybridization, extension and denaturation of the dumbbell-structure oligonucleotide.

According to the present invention, the amplification reaction may be carried out through PCR.

A variety of DNA polymerases may be used in the PCR of the present invention, and may include "Klenow" fragments of *E. coli* DNA polymerase I, thermostable DNA polymerase and bacteriophage T7 DNA polymerase. Preferably, the polymerase is a thermostable DNA polymerase obtainable from a variety of bacterial species, and includes *Thermus aquaticus* (Taq), *Thermus thermophilus* (Tth), *Thermus filiformis, Thermus flavus, Thermococcus litoralis,* and *Pyrococcus furiosus* (Pfu). When the polymerization reaction is carried out, the reaction vessel is preferably supplied with an excess of the components necessary for the reaction. An excess of components required for the amplification reaction may mean an amount such that the amplification reaction is not substantially restricted by the component concentration. It is desirable to supply a cofactor such as Mg²⁺, dATP, dCTP, dGTP and dTTP to the reaction mixture such that the desired degree of amplification may be achieved.

In an embodiment of the present invention, the primary amplification in step (a) may be performed to a time point before serious competition between the amplification products occurs.

According to the present invention, the fluorogenic reporter in step (b) is SYBR Green, SYTO 9, EvaGreen or LCGreen.

Described and claimed *per se* is that the primer used in step (b) may be a conventional primer, or a different dumbbell-structure oligonucleotide. According to the present invention, the primer used in step (b) is the same dumbbell-structure oligonucleotide as the oligonucleotide used in step (a).

According to the present invention, the dumbbell-structure oligonucleotides used in steps (a) and (b) is the same as each other, and in step (b), oligonucleotide priming may preferentially occur using a primer having high specificity.

In an embodiment of the present invention, at least one of the oligonucleotides used in steps (a) and (b) includes a UTP nucleotide, and may thus be decomposed by the UNG enzyme.

In an embodiment of the present invention, at least one of the oligonucleotides used in step (a) includes a UTP nucleotide, and after step (a), the oligonucleotide is removed by the UNG enzyme, thus making it possible to substantially prevent contamination in secondary amplification due to the primer.

In an embodiment of the present invention, step (a) may be performed in the range of 1 to 25 cycles. In another embodiment of the present invention, step (a) may be performed in the range of 5 to 20 cycles. In still another embodiment of the present invention, step (a) may be performed in the range of 10 to 15 cycles. Here, the range of cycles may be freely altered by those skilled in the art.

In an embodiment of the present invention, the signal detection in step (b) may be performed by measuring the fluorescence signal generated from the fluorogenic reporter every cycle and plotting it on the amplification curve. For example, a predetermined threshold is applied to the amplification curve, thereby evaluating whether the target nucleic acid sequence is present or absent. Also, the amplification curve may be used for quantitative analysis of the target nucleic acid sequence. For example, the amount of target nucleic acid sequence present in the sample may be determined by measuring the Ct (Cycle threshold) value on the amplification curve. The qualitative and quantitative analysis of the target nucleic acid sequences is known in the art.

According to the present invention, the signal detection in step (b) is performed through melting curve analysis. Melting curve analysis is well known in the art. For example, after completion of step (b), a melting curve may be plotted by measuring fluorescence while elevating the temperature of the final product at constant temperature intervals. According to the present invention, the melting curve is generated with a resolution ranging from 0.05°C to 0.02°C. Described and not claimed *per se* is that the melting curve may be generated with a resolution of less than 0.02°C.

In the conventional real-time PCR method, since amplification and detection have to be performed at the same time, high throughput thereof is limited. For example, the conventional real-time PCR takes about 2 hr to detect because amplification and detection are simultaneously conducted. However, since amplification and detection may be separately performed in the present invention, detection results may be obtained within 30 min using a real-time PCR device.

Also, the dumbbell-structure oligonucleotide is used in the present invention, whereby the use of a fluorescent dual-labeled probe may be decreased, thus significantly reducing the cost of analysis, and furthermore, the amount of the sample to be used and the analysis time may be reduced, and the detection sensitivity may be greatly improved.

Moreover, the multiple amplification nested signal amplification (MANSA) technique of the present invention requires a small amount of work in the laboratory because the reaction time is short and the reaction product may be monitored in real time, and the multiplex PCR is able to confirm the presence of multiple gene mutations at the same time, and also to eliminate errors of trace samples that may occur due to false negatives in the conventional real-time PCR.

The present invention pertains to a method of simultaneously detecting various nucleotide mutations or single nucleotide polymorphisms (SNPs) in a nucleotide sequence. In particular, the present invention pertains to a method of simultaneously detecting mutations in the same base or the same codon in a nucleotide sequence.

A method capable of simultaneously detecting two or more nucleotide mutations, particularly, two or more mutations in the same base or the same codon, by a simple amplification reaction (e.g. PCR), has not yet been realized in practice. The present invention addresses a practical method for simultaneous mutation detection using only a simple amplification reaction.

Since hybridization is determined in a type-specific manner by the 5'-low Tm specificity portion and the 3'-high Tm specificity portion separated by the separation portion, the dumbbell-structure oligonucleotide used in the present invention may exhibit greatly improved hybridization specificity.

The 3'-high Tm specificity portion of the dumbbell-structure oligonucleotide of the present invention includes a nucleotide corresponding or complementary to nucleotide mutation. When the oligonucleotide of the present invention is hybridized with the sense strand of the target nucleotide sequence, the 3'-high Tm specificity portion includes a nucleotide complementary to nucleotide mutation, and when it is hybridized with the antisense strand, the 3'-high Tm specificity portion includes a nucleotide corresponding to nucleotide mutation.

In a preferred embodiment of the present invention, the nucleotide corresponding or complementary to nucleotide mutation is located at a position of the 3'-end of the 3'-high Tm specificity portion or a position spaced 1 to 2 bases apart from the 3'-end thereof, and more preferably is located at a position spaced 1 to 2 bases apart from the 3'-end of the 3'-high Tm specificity portion. Most preferably, the nucleotide corresponding or complementary to nucleotide mutation is located at the center of the 3'-high Tm specificity portion or around the center thereof. For example, when the 3'-high Tm specificity portion is spaced 1 to 2 bases apart from the 3'-end, the nucleotide corresponding or complementary to nucleotide mutation is located at a position spaced 1 to 2 bases apart from the 5'-end of the 3'-high Tm specificity portion.

In the 3'-high Tm specificity portion of the oligonucleotide, when the nucleotide corresponding to nucleotide mutation is located at a position spaced 1 to 2 bases apart from the 3'-end of the 3'-high Tm specificity portion and also when the nucleotide complementary to nucleotide mutation is located at a position spaced 1 to 2 bases apart from the 5'-end of the 3'-high Tm specificity portion, the following favorable effects are generated.

For example, when SNP is detected using a conventional primer, the mutation site is located at the 3'-end. As such, the Tm values do not differ significantly based on whether or not (i.e. upon mismatching) the 3'-end base is annealed to the target sequence. Thus, even upon mismatching, there is a strong tendency to obtain false-positive results due to the amplification reaction through annealing. On the other hand, in the case where the mutation site is centered, the Tm values differ somewhat based on whether or not (i.e. upon mismatching) the mutation site is annealed to the target sequence, but the thermostable polymerase mostly catalyzes the polymerization reaction even at the mismatched site, leading to false-positive results.

According to the present invention, the above-described conventional problems may be completely overcome. For example, in the case where the nucleotide corresponding or complementary to nucleotide mutation is located at the center of the 3'-high Tm specificity portion, if mismatching occurs at this position, the Tm value of the 3'-high Tm specificity portion is greatly reduced compared with the case of matching because of mismatching at the center of the structure when viewed only with the 3'-high Tm specificity portion. Because of mismatching at both the 5'-end and the 3'-end when viewed with the overall structure of the primer, the thermostable polymerase does not catalyze the polymerization reaction. Accordingly, false-positive results are not generated upon mismatching.

Another aspect of the present invention pertains to a method of detecting a target nucleic acid sequence using a primer pair including a dumbbell-structure oligonucleotide according to claim 2, comprising the following steps of:

(a) subjecting a target nucleic acid sequence to primary amplification using a primer pair including at least one dumbbell-structure oligonucleotide represented by General Formula below, the primary amplification being performed in a range of 5 to 20 cycles through a primary amplification reaction of a polymerase chain reaction in which a template-dependent extension reaction process, including hybridization, primer extension and denaturation, is repeated using the dumbbell-structure oligonucleotide:
General Formula: 5'-A-B-C-3'
wherein A is a 5'-low Tm specificity portion having a nucleotide sequence that is complementary to one position of the target nucleic acid sequence to be hybridized, and includes a nucleotide sequence complementary to one position of C, B is a separation portion including a contiguous sequence of nucleotides having at least three deoxyinosines as a universal base, C is a 3'-high Tm specificity portion having a nucleotide sequence that is complementary to one position of the target nucleic acid sequence to be hybridized, and includes a nucleotide sequence complementary to one position of A, Tm of A 10 °C to 30 °C and is lower than Tm of C that is 50 °C to 65 °C, Tm of the separation portion is 3 °C to 10 °C that is the lowest among three portions, the separation portion forms a non-base pair structure under the condition that A and C are hybridized to the target nucleic acid sequence, the 5'-low Tm specificity portion is shorter than the 3'-high Tm specificity portion, the 5'-low Tm specificity portion has a length of 3 to 5 nucleotides, the separation portion has a length of 3 to 5 nucleotides, the 3'-high Tm specificity portion has a length of 18 to 30 nucleotides, and the 3'-high Tm specificity portion has a nucleotide sequence fully complementary to the target nucleic acid sequence to be hybridized, hybridization being carried out under the condition that hybridization by C alone does not occur; and
(b) amplifying the primary amplification product through a secondary amplification reaction using a primer pair including an oligonucleotide with the same dumbbell-structure as used in step (a) and a fluorogenic reporter including SYBR Green, SYTO 9, EvaGreen, or LCGreen and detecting a signal from the fluorogenic reporter, in which detection of the signal indicates the presence of the target nucleic acid sequence by analysis of a melting curve generated with a resolution ranging from 0.05 °C to 0.02 °C.

The method of the present invention may be applied to the detection of multiple target nucleic acid sequences.

Still another aspect of the present invention pertains to a method of detecting target nucleic acid sequences using two or more primer pairs including a dumbbell-structure oligonucleotide according to claim 3, comprising the following steps of:
(a) subjecting two or more target nucleic acid sequences to primary amplification using two or more primer pairs including at least one dumbbell-structure oligonucleotide represented by General Formula below, the primary amplification being performed in a range of 5 to 20 cycles through a primary amplification reaction of a polymerase chain reaction in which a template-dependent extension reaction process, including hybridization, primer extension and denaturation, is repeated using the dumbbell-structure oligonucleotide:
   General Formula: 5'-A-B-C-3'
   wherein A is a 5'-low Tm specificity portion having a nucleotide sequence that is complementary to one position of the target nucleic acid sequence to be hybridized, and includes a nucleotide sequence complementary to one position of C, B is a separation portion including a contiguous sequence of nucleotides having at least three deoxyinosines as a universal base, C is a 3'-high Tm specificity portion having a nucleotide sequence that is complementary to one position of the target nucleic acid sequence to be hybridized, and includes a nucleotide sequence complementary to one position of A, Tm of A 10 °C to 30 °C and is lower than Tm of C that is 50 °C to 65 °C, Tm of the separation portion is 3 °C to 10 °C that is the lowest among three portions, the separation portion forms a non-base pair structure under the condition that A and C are hybridized to the target nucleic acid sequence, the 5'-low Tm specificity portion is shorter than the 3'-high Tm specificity portion, the 5'-low Tm specificity portion has a length of 3 to 5 nucleotides, the separation portion has a length of 3 to 5 nucleotides, the 3'-high Tm specificity portion has a length of 18 to 30 nucleotides, and the 3'-high Tm specificity portion has a nucleotide sequence fully complementary to the target nucleic acid sequence to be hybridized, hybridization being carried out under the condition that hybridization by C alone does not occur; and
(b) amplifying the primary amplification product through a secondary amplification reaction using two or more primer pairs including an oligonucleotide with the same dumbbell-structure as used in step (a) and a fluorogenic reporter and detecting a signal from the fluorogenic reporter including SYBR Green, SYTO 9, EvaGreen, or LCGreen and detecting a signal from the fluorogenic reporter, in which detection of the signal indicates the presence of the target nucleic acid sequence by analysis of a melting curve generated with a resolution ranging from 0.05 °C to 0.02 °C.

In an embodiment of the present invention, the oligonucleotides used in steps (a) and (b) may be oligonucleotides for amplifying five or more target nucleic acid sequences, for example, five primer pairs (forward and reverse) and/or probes.

The aforementioned two embodiments differ in the numbers of oligonucleotides and target nucleic acid sequences used and the overall construction thereof is similar to the first embodiment, and thus a detailed description thereof will be omitted.

The present invention is characterized in that:
First, unlike the conventional real-time PCR, according to the method of the present invention, a signal of a target nucleic acid is detected using an amplification product, whereby effective target nucleic acid detection is possible even with Taq at a scale several tenths or hundredths that of the conventional method. Therefore, cheaper products than conventional real-time PCR products may be supplied.

Second, in a conventional testing method, a sample for use in each test is separately required in order to test infection with a pathogen such as a virus or bacteria. Particularly, a sample for a virus infection test, a sample for a bacterial infection test and a sample for a fungal infection test are respectively required. In contrast, in the present invention, target nucleic acid sequences of pathogens, such as 10 or more species of viruses, bacteria and fungi, are simultaneously amplified in a single reaction tube, after which the resulting amplification product may be used to detect target nucleic acid sequences through individual tests on viruses, bacteria and fungi, or diverse combinations of virus/bacteria, virus/bacteria/fungus, and the like. Briefly, it is possible to perform various tests by sampling once and using a small amount of a sample.

Third, the conventional real-time PCR is dependent on the initial target nucleic acid sequence concentration because amplification and detection are performed at the same time using a small amount of target nucleic acid sequence. On the other hand, according to the present invention, amplification of the signal of the target nucleic acid is added during the secondary detection process using the primary amplification product, thereby exhibiting high sensitivity compared to the conventional method. Briefly, according to the present invention, a sensitivity improvement effect similar to that of nested PCR may be achieved.

Fourth, in the present invention, the target nucleic acid sequences of multiple pathogens such as viruses, bacteria and fungi are simultaneously amplified in a single reaction tube, the resulting amplification product is used to perform primary screening (classification), and the pathogen identified as positive for infection may be subjected to secondary identification (individual distinction) using the same amplification product. Particularly, a screening test to check for infection with viruses, bacteria, or fungi in the primary test may be performed, and if the virus is identified as positive for infection, the infected virus may be identified easily and quickly without additional amplification reaction. However, in the conventional method, even if a virus is identified as positive for infection through screening, viral nucleic acid sequence amplification must be separately repeated. Therefore, it is possible to perform various tests using the amplification product through a single amplification process, thereby reducing the time and cost of tests necessary for additional amplification.

Fifth, in the present invention, the target nucleic acid sequence amplification is performed using a general amplification device, and the detection process is performed within 30 min using a real-time detection device. Accordingly, when amplification products are prepared using a general amplification device, 96 samples (when the real-time detection device is a 96-well device) may be tested every 30 min. However, the conventional real-time PCR method requires a minimum of 2 hr and 30 min for every batch of 96 samples.

A multiple amplification nested signal amplification (MANSA) technique of the present invention having this advantage will be described in detail through the following examples.

### Example 1: Detection of target nucleic acid sequence through the method of the present invention

Zika virus genomic DNA was used as a target nucleic acid sequence. The forward and reverse primers having a dumbbell structure were designed based on the Zika virus genomic DNA.

In the present invention, the primer having a dumbbell structure, as represented by General Formula I, has very high specificity and is thus hybridized with the target nucleic acid sequence. In the primer having the structure of General Formula I, the 5'-low Tm specificity portion and the 3'-high Tm specificity portion are physically and functionally separated by the separation portion, and the hybridization specificity of the overall structure of the primer is doubly regulated by the 5'-low Tm specificity portion and the 3'-high Tm specificity portion. Also, the base, corresponding to nucleotide mutation or hybridized thereto, was located at the center of the 3'-high Tm specificity portion, whereby a difference in Tm value due to mismatching was increased, but DNA synthesis by a Taq polymerase was prevented upon mismatching. The target nucleic acid sequences and the primer sequences are shown in Table 1 below.

**[Table 1]**

| | | |
|---|---|---|
| Target | | SEQ ID |
| nucleic acid sequence | | NO:1 |
| Forward Primer | 5'-GCAATIII GGTCATGATACTGCTGATTGC-3' | SEQ ID NO:2 |
| Reverse primer | 5'-GCAATIII CCACAAAGTCCCTATTGC-3' | SEQ ID NO:3 |

The target DNA (final concentration: 1 ng, 10 pg, 1 pg, 100 fg, 10 fg, 1 fg, 100 ag and 10 ag) at various concentrations was placed in a thermocycler (ABI 9700, Perkinelmer) to achieve a final volume of 20 µL containing 5 pmol of a primer and 10 µL of Type-it HRM master mix. The reaction mixture was denatured at 95°C for 10 min and a reaction procedure at 95°C for 30 sec, at 65°C for 60 sec and at 72°C for 60 sec was repeated 15 times, and the reaction was terminated after a final reaction at 72°C for 5 min.

The tube containing the resulting reaction mixture was then placed in a real-time thermocycler (Rotogene Q, Qiagen). The reaction mixture was denatured at 95°C for 10 min, and the reaction procedure at 95°C for 10 sec, at 65°C for 15 sec and at 72°C for 25 sec was repeated 30 times, whereby an increase in the final fluorescence signal was detected. A signal generated in each cycle by a predetermined time interval was detected.

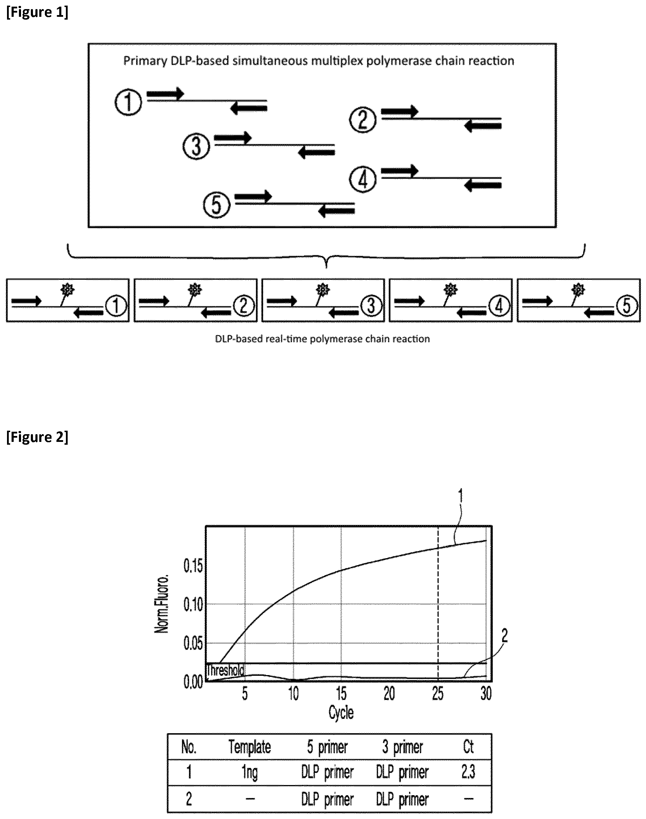

These results are shown in FIG. 2.

As shown in FIG. 2, the fluorescence signal amplification of the target nucleic acid sequence did not occur in a very small amount of the template, indicating that signal amplification does not occur by hybridization of the target template with the primers of the present invention or by cleavage of the single-stranded primer of the present invention alone. On the other hand, fluorescence signals of target nucleic acid sequences up to 1 fg were confirmed in the testing of the target nucleic acid sequence at different concentrations. Thus, without amplification of the target nucleic acid sequence, it is sufficient to repeat denaturation, hybridization, cleavage and extension using the primers of the present invention in order to generate the target fluorescence signal, thereby making it possible to detect the target nucleic acid sequence by real-time signal amplification.

### Example 2: PCR sensitivity of the method of the present invention

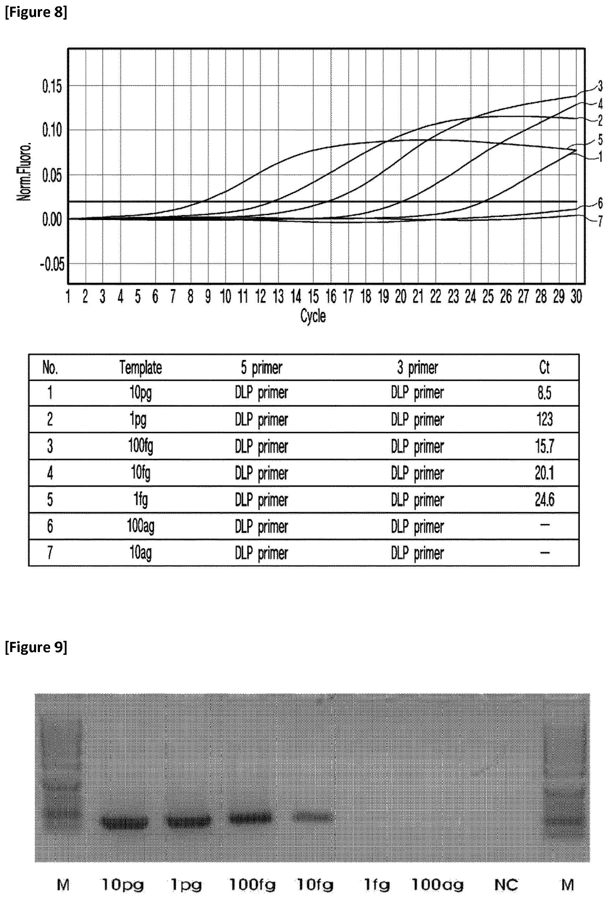

Real-time PCR sensitivity using the primers of the present invention was confirmed by detecting the target nucleic acid sequence of the Zika virus gene. As shown in FIG. 8, when real-time PCR was performed using Zika virus genomic DNA diluted serially starting from 1 ng, the target nucleic acid sequence was detected even upon dilution up to 10 fg.

The results of electrophoretic analysis of the amplification product are shown in FIG. 9. As shown in FIG. 9, it was confirmed that the amplification product was generated in proportion to the initial target nucleic acid sequence concentration.

### Example 3: Melting temperature curve analysis using primer of the present invention

When denaturation, hybridization, cleavage and extension were repeated at different DNA concentrations under real-time PCR reaction conditions, whether the primers of the present invention could generate enough signal to detect the target nucleic acid sequence was additionally evaluated.

To perform this evaluation, the same template and primers used in Example 1 were used for real-time target signal amplification. The DNA (final concentration: 1 ng, 10 pg, 1 pg, 100 fg, 10 fg, 1 fg, 100 ag and 10 ag) at various concentrations, having a final volume of 20 µL containing 5 pmol of a primer and 10 µL of Type-it HRM master mix, was subjected to real-time target signal amplification. The tube containing the resulting reaction mixture was placed in a real-time thermocycler (Rotogene Q, Qiagen). 1 µL of each PCR product thus obtained, serving as a test sample, was subjected to real-time PCR using a Type-it HRM PCR kit (Qiagen Inc., Germantown, MD, USA). About 10 µL of 2X Type-it HRM PCR master mix, 0.1 µL of APC primer mixed solution, and 9 µL of distilled water were added thereto, followed by real-time PCR. The Type-it HRM PCR master mix contained EvaGreen as a fluorescence pigment.

The real-time PCR was carried out at 95°C for 10 min using a Rotor-gene Q (Qiagen Inc., Germantown, MD, USA), after which the reaction procedure at 95°C for 10 sec, at 65°C for 15 sec and 72°C for 20 sec was repeated 30 times. After completion of the final PCR, melting curve analysis was performed by measuring the fluorescence wavelength at 510 nm while the temperature was elevated from about 77°C to 89°C at a rate of about 0.2 °C/sec, whereby changes in fluorescence intensity of the amplification product were observed.

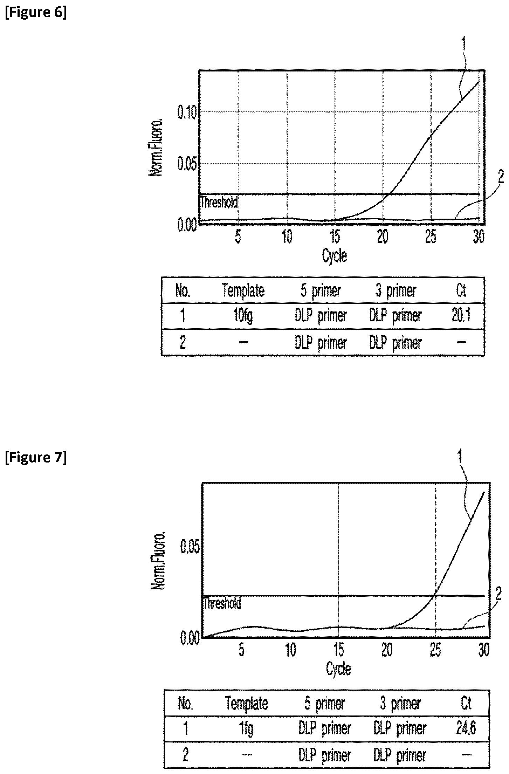

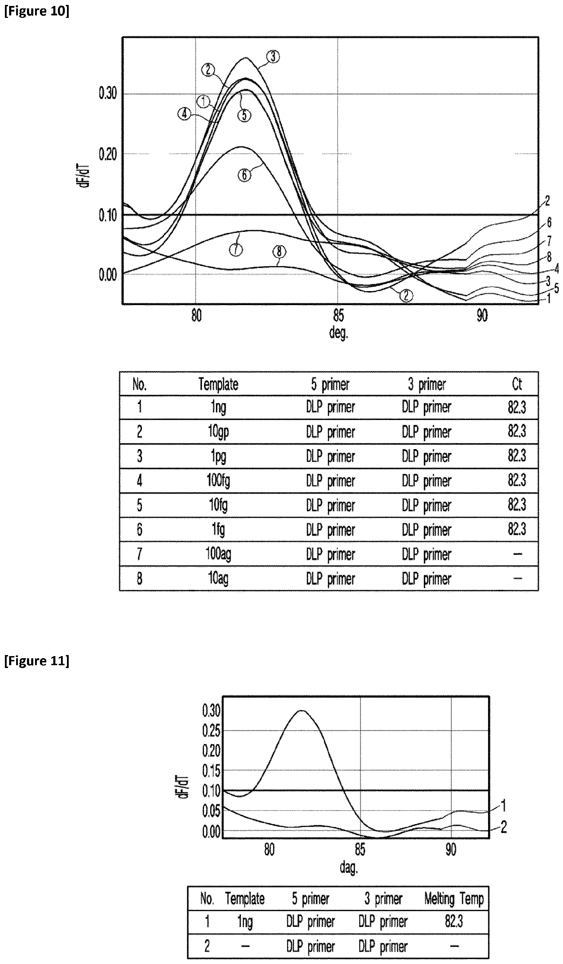

As shown in FIG. 10, no fluorescence signal amplification of the target nucleic acid sequence was observed in a very small amount of template and thus the melting temperature curve was not observed, indicating that signal amplification does not occur by hybridization of the target template with the primers of the present invention or by cleavage of the single-stranded primer of the present invention alone. On the other hand, the fluorescence signal of the target nucleic acid sequence up to 1 fg was confirmed by the template concentration, and the melting temperature curve was confirmed based thereon. Furthermore, as shown in FIG. 6, the melting temperature curve analysis by the template concentration made it possible to analyze the melting temperature curve in the target nucleic acid sequence up to 1 fg.
## Claims

1. A method of detecting a target nucleic acid sequence using a dumbbell-structure oligonucleotide, comprising:
(a) amplifying a target nucleic acid sequence through a primary amplification reaction of a polymerase chain reaction in which a primer pair having a dumbbell-structure oligonucleotide represented by General Formula below is used, annealing is carried out in a temperature range of 50 °C to 65 °C, and a template-dependent extension reaction process, including hybridization, extension, and denaturation using the dumbbell-structure oligonucleotide, is repeated:
General Formula: 5'-A-B-C-3'
wherein A is a 5'-low Tm specificity portion having a nucleotide sequence that is complementary to one position of the target nucleic acid sequence to be hybridized, and includes a nucleotide sequence complementary to one position of C, B is a separation portion including at least three universal bases, C is a 3'-high Tm specificity portion having a nucleotide sequence that is complementary to one position of the target nucleic acid sequence to be hybridized, and includes a nucleotide sequence complementary to one position of A, Tm of A is 10 °C to 30 °C and is lower than Tm of C that is 50 °C to 65 °C, Tm of the separation portion is 3 °C to 10 °C that is the lowest among three portions, the separation portion forms a non-base pair structure under a condition that A and C are hybridized to the target nucleic acid sequence, the 5'-low Tm specificity portion is shorter than the 3'-high Tm specificity portion, the 5'-low Tm specificity portion has a length of 3 to 5 nucleotides, the separation portion has a length of 3 to 5 nucleotides, the 3'-high Tm specificity portion has a length of 18 to 30 nucleotides, and the 3'-high Tm specificity portion has a nucleotide sequence fully complementary to the target nucleic acid sequence to be hybridized, hybridization being carried out under a condition that hybridization by C alone does not occur; and
(b) amplifying a primary amplification product through a secondary amplification reaction using a primer pair including an oligonucleotide with the same dumbbell-structure as used in step (a)and a fluorogenic reporter including SYBR Green, SYTO 9, EvaGreen, or LCGreen and detecting a signal from the fluorogenic reporter, in which detection of the signal indicates presence of the target nucleic acid sequence by analysis of a melting curve generated with a resolution ranging from 0.05 °C to 0.02 °C.

2. A method of detecting a target nucleic acid sequence using a dumbbell-structure oligonucleotide, comprising:
(a) subjecting a target nucleic acid sequence to primary amplification using a primer pair including at least one dumbbell-structure oligonucleotide represented by General Formula below, the primary amplification being performed in a range of 5 to 20 cycles through a primary amplification reaction of a polymerase chain reaction in which a template-dependent extension reaction process, including hybridization, extension, and denaturation, is repeated using the dumbbell-structure oligonucleotide:
General Formula: 5'-A-B-C-3'
wherein A is a 5'-low Tm specificity portion having a nucleotide sequence that is complementary to one position of the target nucleic acid sequence to be hybridized, and includes a nucleotide sequence complementary to one position of C, B is a separation portion including a contiguous sequence of nucleotides having at least three deoxyinosines as a universal base, C is a 3'-high Tm specificity portion having a nucleotide sequence that is complementary to one position of the target nucleic acid sequence to be hybridized, and includes a nucleotide sequence complementary to one position of A, Tm of A is 10 °C to 30 °C and is lower than Tm of C that is 50 °C to 65 °C, Tm of the separation portion is 3 °C to 10 °C that is the lowest among three portions, the separation portion forms a non-base pair structure under a condition that A and C are hybridized to the target nucleic acid sequence, the 5'-low Tm specificity portion is shorter than the 3'-high Tm specificity portion, the 5'-low Tm specificity portion has a length of 3 to 5 nucleotides, the separation portion has a length of 3 to 5 nucleotides, the 3'-high Tm specificity portion has a length of 18 to 30 nucleotides, and the 3'-high Tm specificity portion has a nucleotide sequence fully complementary to the target nucleic acid sequence to be hybridized, hybridization being carried out under a condition that hybridization by C alone does not occur; and
(b) amplifying a primary amplification product through a secondary amplification reaction using a primer pair including an oligonucleotide with the same dumbbell-structure as used in step (a) and a fluorogenic reporter including SYBR Green, SYTO 9, EvaGreen, or LCGreen and detecting a signal from the fluorogenic reporter, in which detection of the signal indicates presence of the target nucleic acid sequence by analysis of a melting curve generated with a resolution ranging from 0.05 °C to 0.02 °C.

3. A method of detecting two or more target nucleic acid sequences using a dumbbell-structure oligonucleotide, comprising:
(a) subjecting two or more target nucleic acid sequences to primary amplification using two or more primer pairs including at least one dumbbell-structure oligonucleotide represented by General Formula below, the primary amplification being performed in a range of 5 to 20 cycles through a primary amplification reaction of a polymerase chain reaction in which a template-dependent extension reaction process, including hybridization, extension, and denaturation, is repeated using the dumbbell-structure oligonucleotide:
General Formula: 5'-A-B-C-3'
wherein A is a 5'-low Tm specificity portion having a nucleotide sequence that is complementary to one position of the target nucleic acid sequence to be hybridized, and includes a nucleotide sequence complementary to one position of C, B is a separation portion including a contiguous sequence of nucleotides having at least three deoxyinosines as a universal base, C is a 3'-high Tm specificity portion having a nucleotide sequence that is complementary to one position of the target nucleic acid sequence to be hybridized, and includes a nucleotide sequence complementary to one position of A, Tm of A is 10 °C to 30 °C and is lower than Tm of C that is 50 °C to 65 °C, Tm of the separation portion is 3 °C to 10 °C that is the lowest among three portions, the separation portion forms a non-base pair structure under a condition that A and C are hybridized to the target nucleic acid sequence, the 5'-low Tm specificity portion is shorter than the 3'-high Tm specificity portion, the 5'-low Tm specificity portion has a length of 3 to 5 nucleotides, the separation portion has a length of 3 to 5 nucleotides, the 3'-high Tm specificity portion has a length of 18 to 30 nucleotides, and the 3'-high Tm specificity portion has a nucleotide sequence fully complementary to the target nucleic acid sequence to be hybridized, hybridization being carried out under a condition that hybridization by C alone does not occur; and
(b) amplifying a primary amplification product through a secondary amplification reaction using two or more primer pairs including an oligonucleotide with the same dumbbell-structure as used in step (a) and a fluorogenic reporter including SYBR Green, SYTO 9, EvaGreen, or LCGreen and detecting a signal from the fluorogenic reporter, in which detection of the signal indicates presence of the target nucleic acid sequence by analysis of a melting curve generated with a resolution ranging from 0.05 °C to 0.02 °C.

4. The method of any one of claims 1 to 3, wherein the primary amplification in step (a) is performed up to a time point before serious competition between amplification products occurs.

## Patentansprüche

1. Verfahren zum Erfassen einer Zielnukleinsäuresequenz unter Verwendung eines Oligonukleotids mit Hantelstruktur, umfassend:
(a) Amplifizieren einer Zielnukleinsäuresequenz durch eine primäre Amplifikationsreaktion einer Polymerase-Kettenreaktion, in der ein Primerpaar mit einem Oligonukleotid mit Hantelstruktur, das durch die Allgemeine Formel im Folgenden dargestellt ist, verwendet wird, wobei ein Annealing in einem Temperaturbereich von 50°C bis 65°C ausgeführt wird und ein Matrizen-abhängiger Extensionsreaktionsvorgang, einschließlich Hybridisierung, Extension und Denaturierung, unter Verwendung des Oligonukleotids mit Hantelstruktur wiederholt wird:
Allgemeine Formel: 5'-A-B-C-3'
wobei A ein Nieder-Tm-5'-Spezifizitätsabschnitt mit einer Nukleotidsequenz ist, die komplementär zu einer Position der zu hybridisierenden Zielnukleinsäuresequenz ist, und eine Nukleotidsequenz aufweist, die komplementär zu einer Position von C ist, wobei B ein Trennungsabschnitt ist, der mindestens drei universelle Basen aufweist,
wobei C ein Hoch-Tm-3'-Spezifizitätsabschnitt mit einer Nukleotidsequenz ist, die komplementär zu einer Position der zu hybridisierenden Zielnukleinsäuresequenz ist, und eine Nukleotidsequenz aufweist, die komplementär zu einer Position von A ist, wobei Tm von A 10 °C bis 30 °C beträgt und niedriger ist als Tm von C, die 50 °C bis 65 °C beträgt, wobei Tm des Trennungsabschnitts 3 °C bis 10 °C beträgt, welche die niedrigste unter drei Abschnitten ist, wobei der Trennungsabschnitt eine Nicht-Basenpaar-Struktur unter einer Bedingung bildet, dass A und C zu der Zielnukleinsäuresequenz hybridisiert werden, wobei der Nieder-Tm-5'-Spezifizitätsabschnitt kürzer ist als der Hoch-Tm-3'-Spezifizitätsabschnitt, wobei der Nieder-Tm-5'-Spezifizitätsabschnitt eine Länge von 3 bis 5 Nukleotiden aufweist, wobei der Trennungsabschnitt eine Länge von 3 bis 5 Nukleotiden aufweist, wobei der Hoch-Tm-3'-Spezifizitätsabschnitt eine Länge von 18 bis 30 Nukleotiden aufweist und wobei der Hoch-Tm-3'-Spezifizitätsabschnitteine eine Nukleotidsequenz aufweist, die vollständig komplementär zu der zu hybridisierenden Zielnukleinsäuresequenz ist, wobei eine Hybridisierung unter einer Bedingung ausgeführt wird, dass eine Hybridisierung allein durch C nicht stattfindet; und
(b) Amplifizieren eines primären Amplifikationsprodukts durch eine sekundäre Amplifikationsreaktion unter Verwendung eines Primerpaares, das ein Oligonukleotid mit der gleichen Hantelstruktur, wie in Schritt (a) verwendet wird, aufweist, und eines fluorogenen Reporters, der SYBR Green, SYTO 9, EvaGreen oder LCGreen aufweist, und Erfassen eines Signals von dem fluorogenen Reporter, wobei die Erfassung des Signals das Vorhandensein der Zielnukleinsäuresequenz durch eine Analyse einer Schmelzkurve, die mit einer Auflösung in einem Bereich von 0,05 °C bis 0,02 °C erzeugt wird, angibt.

2. Verfahren zum Erfassen einer Zielnukleinsäuresequenz unter Verwendung eines Oligonukleotids mit Hantelstruktur, umfassend:
(a) Unterziehen einer Zielnukleinsäuresequenz einer primären Amplifikation unter Verwendung eines Primerpaares, das mindestens ein Oligonukleotid mit Hantelstruktur aufweist, das durch die Allgemeine Formel im Folgenden dargestellt wird, wobei die primäre Amplifikation in einem Bereich von 5 bis 20 Zyklen durch eine primäre Amplifikationsreaktion einer Polymerase-Kettenreaktion durchgeführt wird, in der ein Matrizen-abhängiger Extensionsreaktionsvorgang, einschließlich Hybridisierung, Extension und Denaturierung, unter Verwendung des Oligonukleotids mit Hantelstruktur wiederholt wird:
Allgemeine Formel: 5'-A-B-C-3'
wobei A ein Nieder-Tm-5'-Spezifizitätsabschnitt mit einer Nukleotidsequenz ist, die komplementär zu einer Position der zu hybridisierenden Zielnukleinsäuresequenz ist, und eine Nukleotidsequenz aufweist, die komplementär zu einer Position von C ist, wobei B ein Trennungsabschnitt ist, der eine fortlaufende Sequenz aus Nukleotiden mit mindestens drei Deoxyinosinen als eine universelle Base aufweist, wobei C ein Hoch-Tm-3'-Spezifizitätsabschnitt mit einer Nukleotidsequenz ist, die komplementär zu einer Position der zu hybridisierenden Zielnukleinsäuresequenz ist, und eine Nukleotidsequenz aufweist, die komplementär zu einer Position von A ist, wobei Tm von A 10 °C bis 30 °C beträgt und niedriger ist als Tm von C, die 50 °C bis 65 °C beträgt, wobei Tm des Trennungsabschnitts 3 °C bis 10 °C beträgt, welche die niedrigste unter drei Abschnitten ist, wobei der Trennungsabschnitt eine Nicht-Basenpaar-Struktur unter einer Bedingung bildet, dass A und C zu der Zielnukleinsäuresequenz hybridisiert werden, wobei der Nieder-Tm-5'-Spezifizitätsabschnitt kürzer ist als der Hoch-Tm-3'-Spezifizitätsabschnitt, wobei der Nieder-Tm-5'-Spezifizitätsabschnitt eine Länge von 3 bis 5 Nukleotiden aufweist, wobei der Trennungsabschnitt eine Länge von 3 bis 5 Nukleotiden aufweist, wobei der Hoch-Tm-3'-Spezifizitätsabschnitt eine Länge von 18 bis 30 Nukleotiden aufweist und wobei der Hoch-Tm-3'-Spezifizitätsabschnitteine eine Nukleotidsequenz aufweist, die vollständig komplementär zu der zu hybridisierenden Zielnukleinsäuresequenz ist, wobei eine Hybridisierung unter einer Bedingung ausgeführt wird, dass eine Hybridisierung allein durch C nicht stattfindet; und
(b) Amplifizieren eines primären Amplifikationsprodukts durch eine sekundäre Amplifikationsreaktion unter Verwendung eines Primerpaares, das ein Oligonukleotid mit der gleichen Hantelstruktur, wie in Schritt (a) verwendet wird, aufweist, und eines fluorogenen Reporters, der SYBR Green, SYTO 9, EvaGreen oder LCGreen aufweist, und Erfassen eines Signals von dem fluorogenen Reporter, wobei die Erfassung des Signals das Vorhandensein der Zielnukleinsäuresequenz durch eine Analyse einer Schmelzkurve, die mit einer Auflösung in einem Bereich von 0,05 °C bis 0,02 °C erzeugt wird, angibt.

3. Verfahren zum Erfassen von zwei oder mehr Zielnukleinsäuresequenzen unter Verwendung eines Oligonukleotids mit Hantelstruktur, umfassend:
(a) Unterziehen von zwei oder mehr Zielnukleinsäuresequenzen einer primären Amplifikation unter Verwendung von zwei oder mehr Primerpaaren, die mindestens ein Oligonukleotid mit Hantelstruktur aufweisen, das durch die Allgemeine Formel im Folgenden dargestellt wird, wobei die primäre Amplifikation in einem Bereich von 5 bis 20 Zyklen durch eine primäre Amplifikationsreaktion einer Polymerase-Kettenreaktion durchgeführt wird, in der ein Matrizen-abhängiger Extensionsreaktionsvorgang, einschließlich Hybridisierung, Extension und Denaturierung, unter Verwendung des Oligonukleotids mit Hantelstruktur wiederholt wird:
Allgemeine Formel: 5'-A-B-C-3'
wobei A ein Nieder-Tm-5'-Spezifizitätsabschnitt mit einer Nukleotidsequenz ist, die komplementär zu einer Position der zu hybridisierenden Zielnukleinsäuresequenz ist, und eine Nukleotidsequenz aufweist, die komplementär zu einer Position von C ist, wobei B ein Trennungsabschnitt ist, der eine fortlaufende Sequenz aus Nukleotiden mit mindestens drei Deoxyinosinen als eine universelle Base aufweist, wobei C ein Hoch-Tm-3'-Spezifizitätsabschnitt mit einer Nukleotidsequenz ist, die komplementär zu einer Position der zu hybridisierenden Zielnukleinsäuresequenz ist, und eine Nukleotidsequenz aufweist, die komplementär zu einer Position von A ist, wobei Tm von A 10 °C bis 30 °C beträgt und niedriger ist als Tm von C, die 50 °C bis 65 °C beträgt, wobei Tm des Trennungsabschnitts 3 °C bis 10 °C beträgt, welche die niedrigste unter drei Abschnitten ist, wobei der Trennungsabschnitt eine Nicht-Basenpaar-Struktur unter einer Bedingung bildet, dass A und C zu der Zielnukleinsäuresequenz hybridisiert werden, wobei der Nieder-Tm-5'-Spezifizitätsabschnitt kürzer ist als der Hoch-Tm-3'-Spezifizitätsabschnitt, wobei der Nieder-Tm-5'-Spezifizitätsabschnitt eine Länge von 3 bis 5 Nukleotiden aufweist, wobei der Trennungsabschnitt eine Länge von 3 bis 5 Nukleotiden aufweist, wobei der Hoch-Tm-3'-Spezifizitätsabschnitt eine Länge von 18 bis 30 Nukleotiden aufweist und wobei der Hoch-Tm-3'-Spezifizitätsabschnitteine eine Nukleotidsequenz aufweist, die vollständig komplementär zu der zu hybridisierenden Zielnukleinsäuresequenz ist, wobei eine Hybridisierung unter einer Bedingung ausgeführt wird, dass eine Hybridisierung allein durch C nicht stattfindet; und
(b) Amplifizieren eines primären Amplifikationsprodukts durch eine sekundäre Amplifikationsreaktion unter Verwendung von zwei oder mehr Primerpaaren, die ein Oligonukleotid mit der gleichen Hantelstruktur, wie in Schritt (a) verwendet wird, aufweisen, und eines fluorogenen Reporters, der SYBR Green, SYTO 9, EvaGreen oder LCGreen aufweist, und Erfassen eines Signals von dem fluorogenen Reporter, wobei die Erfassung des Signals das Vorhandensein der Zielnukleinsäuresequenz durch eine Analyse einer Schmelzkurve, die mit einer Auflösung in einem Bereich von 0,05 °C bis 0,02 °C erzeugt wird, angibt.

4. Verfahren nach einem der Ansprüche 1 bis 3, wobei die primäre Amplifikation in Schritt (a) bis zu einem Zeitpunkt durchgeführt wird, bevor eine ernsthafte Konkurrenz zwischen Amplifikationsprodukten stattfindet.

## Revendications

1. Procédé de détection d'une séquence d'acide nucléique cible en utilisant un oligonucléotide à structure en haltère, comprenant de :
(a) amplifier une séquence d'acide nucléique cible par une réaction d'amplification primaire d'une réaction en chaîne par polymérase dans laquelle une paire d'amorces ayant un oligonucléotide à structure en haltère représenté par la formule générale ci-dessous est utilisée, un annelage est effectué dans une plage de température de 50 °C à 65 °C, et un processus de réaction d'extension dépendant de la matrice, comprenant l'hybridation, l'extension et la dénaturation en utilisant l'oligonucléotide à structure en haltère, est répété :
Formule générale : 5'-A-B-C-3' où A est une partie de spécificité 5' à faible Tm ayant une séquence de nucléotides qui est complémentaire d'une position de la séquence d'acide nucléique cible à hybrider, et inclut une séquence de nucléotides complémentaire d'une position de C, B est une partie de séparation incluant au moins trois bases universelles, C est une partie de spécificité 3' à Tm élevée ayant une séquence de nucléotides qui est complémentaire d'une position de la séquence d'acide nucléique cible à hybrider, et inclut une séquence de nucléotides complémentaire d'une position de A, la Tm de A est de 10 °C à 30 °C et est inférieure à la Tm de C qui est de 50 °C à 65 °C, la Tm de la partie de séparation est de 3 °C à 10 °C, qui est la plus basse parmi les trois parties, la partie de séparation forme une structure de non-paire de bases dans une condition où A et C sont hybridés à la séquence d'acide nucléique cible, la partie de spécificité 5' à faible Tm est plus courte que la partie de spécificité 3' à Tm élevée, la partie de spécificité 5' à faible Tm a une longueur de 3 à 5 nucléotides, la partie de séparation a une longueur de 3 à 5 nucléotides, la partie de spécificité 3' à Tm élevée a une longueur de 18 à 30 nucléotides, et la partie de spécificité 3' à Tm élevée a une séquence de nucléotides entièrement complémentaire de la séquence d'acide nucléique cible à hybrider, l'hybridation étant effectuée dans une condition telle que l'hybridation par C seul ne se produit pas□; et
(b) amplifier un produit d'amplification primaire par une réaction d'amplification secondaire en utilisant une paire d'amorces incluant un oligonucléotide avec la même structure en haltère que celle utilisée à l'étape (a) et un rapporteur fluorogène incluant le SYBR Green, le SYTO 9, l'EvaGreen ou le LCGreen et détecter un signal à partir du rapporteur fluorogène, dans lequel la détection du signal indique la présence de la séquence d'acide nucléique cible par analyse d'une courbe de fusion générée avec une résolution allant de 0,05 °C à 0,02 °C.

2. Procédé de détection d'une séquence d'acide nucléique cible en utilisant un oligonucléotide à structure en haltère, comprenant de :
(a) soumettre une séquence d'acide nucléique cible à une amplification primaire en utilisant une paire d'amorces incluant au moins un oligonucléotide à structure en haltère représenté par la formule générale ci-dessous, l'amplification primaire étant réalisée dans une plage de 5 à 20 cycles par une réaction d'amplification primaire d'une réaction en chaîne par polymérase dans laquelle un processus de réaction d'extension dépendant de la matrice, incluant une hybridation, une extension et une dénaturation, est répété en utilisant l'oligonucléotide à structure en haltère :
Formule générale : 5'-A-B-C-3'
où A est une partie de spécificité 5' à faible Tm ayant une séquence de nucléotides qui est complémentaire d'une position de la séquence d'acide nucléique cible à hybrider, et inclut une séquence de nucléotides complémentaire d'une position de C, B est une partie de séparation incluant une séquence contiguë de nucléotides ayant au moins trois désoxyinosines comme base universelle, C est une partie de spécificité 3' à Tm élevée ayant une séquence de nucléotides qui est complémentaire d'une position de la séquence d'acide nucléique cible à hybrider, et inclut une séquence de nucléotides complémentaire d'une position de A, la Tm de A est de 10 °C à 30 °C et est inférieure à la Tm de C qui est de 50 °C à 65 °C, la Tm de la partie de séparation est de 3 °C à 10 °C qui est la plus basse parmi les trois parties, la partie de séparation forme une structure de non-paire de bases dans une condition où A et C sont hybridés à la séquence d'acide nucléique cible, la partie de spécificité 5' à faible Tm est plus courte que la partie de spécificité 3' à Tm élevée, la partie de spécificité 5' à faible Tm a une longueur de 3 à 5 nucléotides, la partie de séparation a une longueur de 3 à 5 nucléotides, la partie de spécificité 3' à Tm élevée a une longueur de 18 à 30 nucléotides, et la partie de spécificité 3' à Tm élevée a une séquence de nucléotides entièrement complémentaire de la séquence d'acide nucléique cible à hybrider, l'hybridation étant effectuée dans une condition où l'hybridation par C seul ne se produit pas□; et
(b) amplifier un produit d'amplification primaire par une réaction d'amplification secondaire en utilisant une paire d'amorces incluant un oligonucléotide avec la même structure en haltère que celle utilisée à l'étape (a) et un rapporteur fluorogène incluant le SYBR Green, le SYTO 9, l'EvaGreen ou le LCGreen et détecter un signal à partir du rapporteur fluorogène, dans lequel la détection du signal indique la présence de la séquence d'acide nucléique cible par analyse d'une courbe de fusion générée avec une résolution allant de 0,05 °C à 0,02 °C.

3. Procédé de détection de deux séquences d'acide nucléique cibles en utilisant un oligonucléotide à structure en haltère, comprenant de :
(a) soumettre deux séquences d'acide nucléique cibles ou plus à une amplification primaire en utilisant deux paires d'amorces ou plus incluant au moins un oligonucléotide à structure en haltère représenté par la formule générale ci-dessous, l'amplification primaire étant réalisée dans une plage de 5 à 20 cycles par une réaction d'amplification primaire d'une réaction en chaîne par polymérase dans laquelle un processus de réaction d'extension dépendant de la matrice, incluant une hybridation, une extension et une dénaturation, est répété en utilisant l'oligonucléotide à structure en haltère :
Formule générale : 5'-A-B-C-3'
où A est une partie de spécificité 5' à faible Tm ayant une séquence de nucléotides qui est complémentaire d'une position de la séquence d'acide nucléique cible à hybrider, et inclut une séquence de nucléotides complémentaire d'une position de C, B est une partie de séparation incluant une séquence contiguë de nucléotides ayant au moins trois désoxyinosines comme base universelle, C est une partie de spécificité 3' à Tm élevée ayant une séquence de nucléotides qui est complémentaire d'une position de la séquence d'acide nucléique cible à hybrider, et inclut une séquence de nucléotides complémentaire d'une position de A, la Tm de A est de 10 °C à 30 °C et est inférieure à la Tm de C qui est de 50 °C à 65 °C, la Tm de la partie de séparation est de 3 °C à 10 °C qui est la plus basse parmi les trois parties, la partie de séparation forme une structure de non-paire de bases dans une condition où A et C sont hybridés à la séquence d'acide nucléique cible, la partie de spécificité 5' à faible Tm est plus courte que la partie de spécificité 3' à Tm élevée, la partie de spécificité 5' à faible Tm a une longueur de 3 à 5 nucléotides, la partie de séparation a une longueur de 3 à 5 nucléotides, la partie de spécificité 3' à Tm élevée a une longueur de 18 à 30 nucléotides, et la partie de spécificité 3' à Tm élevée a une séquence de nucléotides entièrement complémentaire de la séquence d'acide nucléique cible à hybrider, l'hybridation étant effectuée dans une condition où l'hybridation par C seul ne se produit pas□; et
(b) amplifier un produit d'amplification primaire par une réaction d'amplification secondaire en utilisant deux paires d'amorces ou plus incluant un oligonucléotide avec la même structure en haltère que celle utilisée à l'étape (a) et un rapporteur fluorogène incluant le SYBR Green, le SYTO 9, l'EvaGreen ou le LCGreen et la détection d'un signal à partir du rapporteur fluorogène, dans lequel la détection du signal indique la présence de la séquence d'acide nucléique cible par l'analyse d'une courbe de fusion générée avec une résolution allant de 0,05 °C à 0,02 °C.

4. Procédé selon l'une quelconque des revendications 1 à 3, dans lequel l'amplification primaire à l'étape (a) est effectuée jusqu'à un moment avant qu'une concurrence sérieuse entre les produits d'amplification ne se produise.
